# EUROPEAN PATENT APPLICATION

(11) **EP 4 678 647 A1**
(43) Date of publication of application: **14.01.2026**
(21) Application number: 24769862.4
(22) Date of filing: 08.03.2024
(51) Int. Cl.: C07J 41/00, A61P 25/28, A61P 25/24, A61K 31/57, A61P 25/00, A61P 25/20, A61P 25/08

(54) **SALT FORM AND POLYCRYSTALLINE FORM OF ALLOPREGNANOLONE DERIVATIVE, PREPARATION METHOD THEREFOR, AND USE THEREOF**

(30) Priority: 10.03.2023 CN 202310230221
(71) Applicant: Nanjing Minova Pharmaceutical Co., Ltd., Nanjing, Jiangsu 210046 (CN)
(72) Inventor: LIU, Fei, Nanjing, Jiangsu 210046 (CN); LI, Xianchao, Nanjing, Jiangsu 210046 (CN); WU, Gang, Nanjing, Jiangsu 210046 (CN); ZHAO, Xin, Nanjing, Jiangsu 210046 (CN); LIN, Chenggang, Nanjing, Jiangsu 210046 (CN); LENG, Chaoqun, Nanjing, Jiangsu 210046 (CN)
(74) Representative: Gille Hrabal Partnerschaftsgesellschaft mbB Patentanwälte
(86) International application number: PCT/CN2024/080711
(87) International publication number: WO 2024/188169

(57) **Abstract**

The present invention provides various salt forms of an allopregnanolone derivative, including hydrochloride, phosphate, L-malate, maleate, p-toluenesulfonate, benzoate and the like. The present invention further provides a plurality of crystal forms and amorphous forms of the hydrochloride of the allopregnanolone derivative, including a crystal form I, a crystal form II, a crystal form III, and a crystal form IV. Both the hydrochloride and the phosphate of the allopregnanolone derivative have good stability, and the preparation method is simple and easy to operate, is suitable for scale-up production, and has good industrial application value.

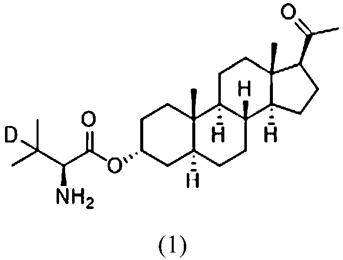

## Description

The present application claims priority to prior Patent Application No. 2023102302217, entitled "SALT FORM AND POLYCRYSTALLINE FORM OF ALLOPREGNANOLONE DERIVATIVE, PREPARATION METHOD THEREFOR, AND USE THEREOF" filed with the China National Intellectual Property Administration on March 10, 2023, the content of which is incorporated herein by reference in its entirety.

### TECHNICAL FIELD

The present disclosure relates to the field of pharmaceutical chemistry, and in particular, to a salt form and polymorphs of an allopregnanolone derivative L-valine-3-d, (3α,5α)-3-hydroxy-pregnan-20-one-3-yl ester (1), a method for preparing same, and use thereof.

### BACKGROUND

In the human body, γ-aminobutyric acid (GABA) is an important inhibitory neurotransmitter, and many nervous system disorders result from changes in the signaling of the neurotransmitter GABA receptor. Mental stress can make the accumulation of GABA in the brain extremely difficult, and the deficiency of GABA may lead to symptoms such as anxiety, fatigue, insomnia, and the like. GABA activates two receptors: the GABA-A receptor subtype and the GABA-B receptor subtype. The GABA-A receptor subtype can modulate neuronal excitability and rapid emotional changes, such as anxiety, panic, and stress responses.

GABA-A receptor subtype positive allosteric modulators (GABA-A PAMs), in particular the neuroactive steroid GABA-A PAMs, have shown clinical efficacy in anesthesia, epilepsy, postpartum depression, and major depression. Currently, the neuroactive steroid that alters the activity of the GABA-A receptor subtype has been systematically investigated as a candidate drug for various nervous system disorders.

Allopregnanolone (3α-OH-5α-pregnan-20-one, also known as brexanolone) is an endogenous pregnane neurosteroid and a positive allosteric modulator of the GABA-A receptor subtype. Studies have shown that allopregnanolone may bind primarily to α and β subunits of the GABA-A receptor subtype, increasing the opening frequency of the chloride ion signal channel on the receptor, decreasing nervous excitability, and thereby producing calming and anti-anxiety effects. However, allopregnanolone has low water solubility and poor oral availability. It has a plasma half-life of about 45 minutes in the human body and can be rapidly metabolized, which is not conducive to making it into oral formulations. In 2019, the U.S. Food and Drug Administration (FDA) approved brexanolone injection for intravenous use (trade name: Zulresso, SAGE Therapeutics) for the treatment of postpartum depression. This drug is the first and only drug currently approved for the treatment of postpartum depression. However, the therapeutic effect of the brexanolone injection can only be achieved by up to 60 hours of intravenous injection, and continuous on-site monitoring and intervention as necessary are required by professional healthcare providers during the infusion, causing great inconvenience to patients and healthcare providers.

In order to overcome the defect, the applicant developed compound 1 shown below, with the chemical name of L-valine-3-d, (3α,5α)-3-hydroxy-pregnan-20-one-3-yl ester:

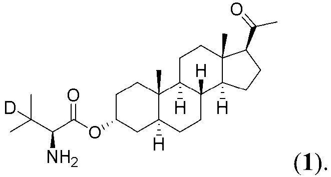

The compound has high oral bioavailability, fast and prolonged action, mild adverse effects, and good metabolic stability, and can be prepared into a suitable oral formulation, thereby possessing improved safety, patient compliance, and convenience.

Different salt forms and crystalline forms of the same drug may have significant differences in appearance, solubility, melting point, dissolution, bioavailability, and the like, thereby affecting the stability, bioavailability, and efficacy of the drug. This phenomenon is particularly pronounced in oral formulations (such as tablets, oral suspensions, and the like). Therefore, further research on the pharmaceutically acceptable salt form and the crystalline form of the compound is of great significance for improving the druggability of the drug.

### SUMMARY

In order to solve the technical problems described above, the present disclosure provides a pharmaceutically acceptable salt of *L*-valine-3-d, (3α,5α)-3-hydroxy-pregnan-20-one-3-yl ester (compound **1**). By salifying the compound, the druggability (such as solubility, stability, hygroscopicity, bioavailability, and the like) thereof is improved, which is beneficial to the development of oral solid formulations.

The present disclosure further provides a polymorph of compound **1** hydrochloride.

The present disclosure further provides a method for preparing a polymorph of compound **1** hydrochloride.

The present disclosure further provides a pharmaceutical composition comprising the pharmaceutically acceptable salt of compound **1** or the crystalline form thereof, and a pharmaceutically acceptable carrier.

The present disclosure further provides use of the pharmaceutically acceptable salt of compound **1** or the crystalline form thereof, or the pharmaceutical composition comprising the pharmaceutically acceptable salt or the crystalline form for manufacturing a medicament for the prevention and/or treatment of central nervous system diseases, Alzheimer's disease, epilepsy, or depression, particularly postpartum depression, or for sedation and hypnosis.

The present disclosure provides a pharmaceutically acceptable salt of compound **1** formed by compound **1** with an organic acid or inorganic acid,

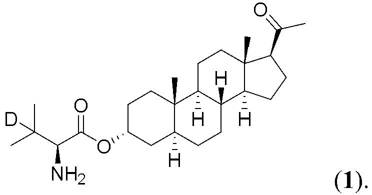

Preferably, the pharmaceutically acceptable salt of compound **1** is selected from hydrochloride, phosphate, benzoate, *p*-toluenesulfonate, malate, acetate, benzenesulfonate, and maleate. More preferably, the pharmaceutically acceptable salt is selected from hydrochloride, phosphate, benzoate, and *p*-toluenesulfonate, further preferably, hydrochloride and phosphate.

In the hydrochloride, benzoate, *p*-toluenesulfonate, acetate, and benzenesulfonate, the molar ratio of compound **1** to the acid is (1±0.1):1, for example, (1±0.05):1, for example, about 1:**1**.

In the phosphate, malate, and maleate, the molar ratio of compound **1** to the acid is (2±0.2):1, for example, (2±0.1):1, for example, about 2:1.

In the present disclosure, the acid includes any optical isomer, racemate, or mesomer thereof. For example, malic acid may be *L*-malic acid, *D*-malic acid, or *D,L*-malic acid.

The present disclosure further provides a polymorph of compound **1** hydrochloride, including: an amorphous form, crystalline forms I, II, III, and IV, wherein compound **1** hydrochloride has a structure shown in the following formula (**2**):

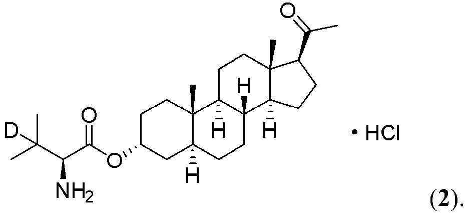

In one embodiment, the crystalline form I of compound **2** has an X-ray powder diffraction pattern comprising characteristic peaks at one or more of 2θ angles of 6.3±0.2°, 10.9±0.2°, 13.3±0.2°, 13.5±0.2°, 15.1+0.2°, and 17.8±0.2°.

In one embodiment, the crystalline form I of compound **2** has an X-ray powder diffraction pattern comprising characteristic peaks at one or more of 2θ angles of 6.3±0.2°, 10.9±0.2°, 13.3+0.2°, 13.5±0.2°, 14.2±0.2°, 15.1±0.2°, 15.8±0.2°, 17.4±0.2°, 17.8±0.2°, 20.9±0.2°, and 24.0±0.2°.

In one embodiment, the crystalline form I has an X-ray powder diffraction pattern comprising diffraction peaks at the following 2θ angles:

| No. | 2θ±0.2° | d value | Relative intensity | No. | 2θ±0.2° | d value | Relative intensity |
|---|---|---|---|---|---|---|---|
| 1 | 6.27 | 14.08 | 14.1% | 7 | 15.12 | 5.85 | 74.3% |
| 2 | 10.90 | 8.11 | 31.8% | 8 | 15.77 | 5.61 | 36.5% |
| 3 | 12.02 | 7.35 | 4.90% | 9 | 17.35 | 5.11 | 24.8% |
| 4 | 13.27 | 6.67 | 54.0% | 10 | 17.78 | 4.98 | 100.0% |
| 5 | 13.49 | 6.56 | 62.2% | 11 | 20.86 | 4.26 | 14.0% |
| 6 | 14.24 | 6.22 | 42.7% | 12 | 23.95 | 3.71 | 16.2% |

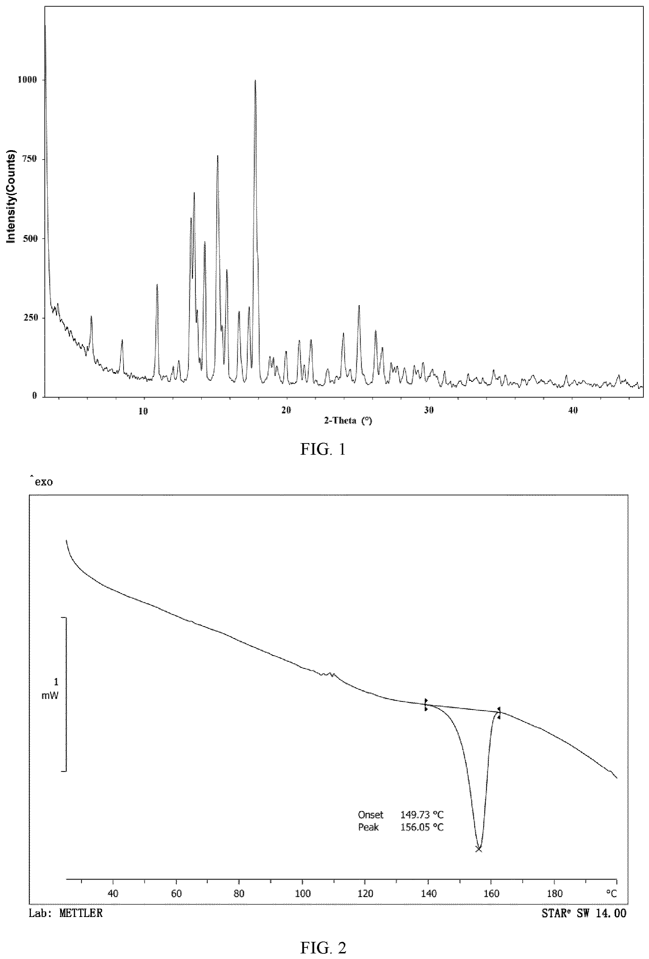

In one embodiment, the crystalline form I has an XRPD pattern substantially as shown in FIG. 1.

In one embodiment, the crystalline form I has a DSC profile comprising an endothermic peak at about 154-158 °C, for example, an endothermic peak at about 156 °C.

In one embodiment, the crystalline form I has a DSC profile substantially as shown in FIG. 2.

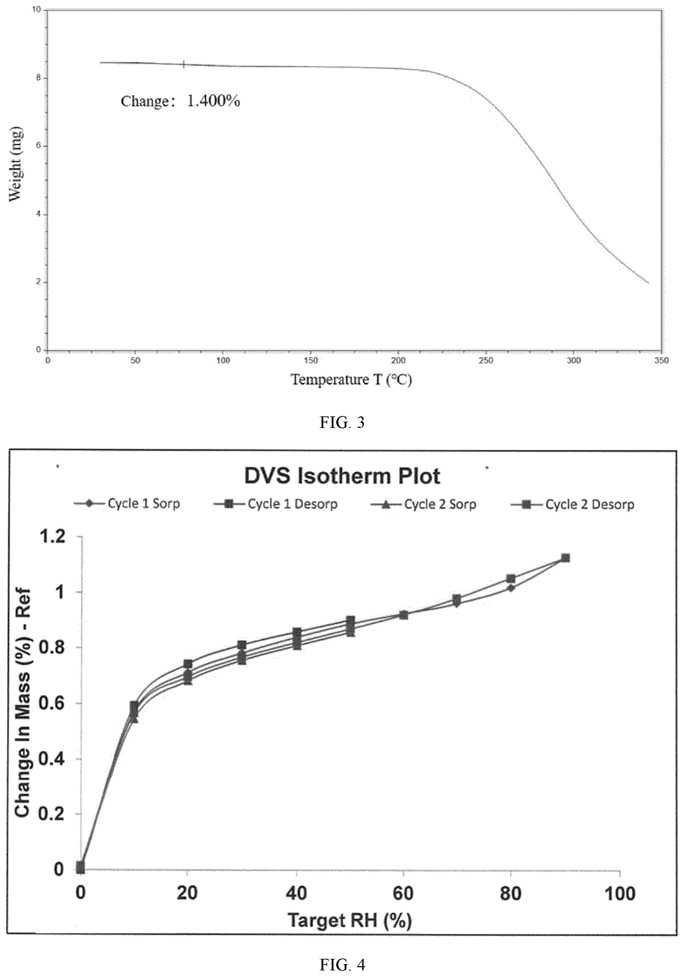

In one embodiment, the crystalline form I has a TGA profile substantially as shown in FIG. 3.

In one embodiment, the crystalline form I has a DVS profile substantially as shown in FIG. 4.

In one embodiment, the crystalline form II of compound 2 has an X-ray powder diffraction pattern comprising characteristic peaks at one or more of 2θ angles of 4.0±0.2°, 10.6±0.2°, 13.9±0.2°, 14.5±0.2°, 18.4±0.2°, and 21.3±0.2°.

In one embodiment, the crystalline form II of compound 2 has an X-ray powder diffraction pattern comprising characteristic peaks at one or more of 2θ angles of 4.0±0.2°, 10.6±0.2°, 13.0±0.2°, 13.9±0.2°, 14.5±0.2°, 18.4±0.2°, and 21.3±0.2°.

In one embodiment, the crystalline form II has an X-ray powder diffraction pattern comprising diffraction peaks at the following 2θ angles:

| No. | 2θ±0.2° | d value | Relative intensity | No. | 2θ+0.2° | d value | Relative intensity |
|---|---|---|---|---|---|---|---|
| 1 | 4.04 | 21.83 | 35.5% | 7 | 14.75 | 6.00 | 23.3% |
| 2 | 8.03 | 11.00 | 18.9% | 8 | 15.30 | 5.79 | 22.6% |
| 3 | 10.61 | 8.33 | 100% | 9 | 15.83 | 5.59 | 15.1% |
| 4 | 13.01 | 6.80 | 34.3% | 10 | 16.80 | 5.27 | 19.4% |
| 5 | 13.90 | 6.37 | 95.3% | 11 | 18.39 | 4.82 | 56.9% |
| 6 | 14.45 | 6.12 | 39.3% | 12 | 21.25 | 4.18 | 37.5% |

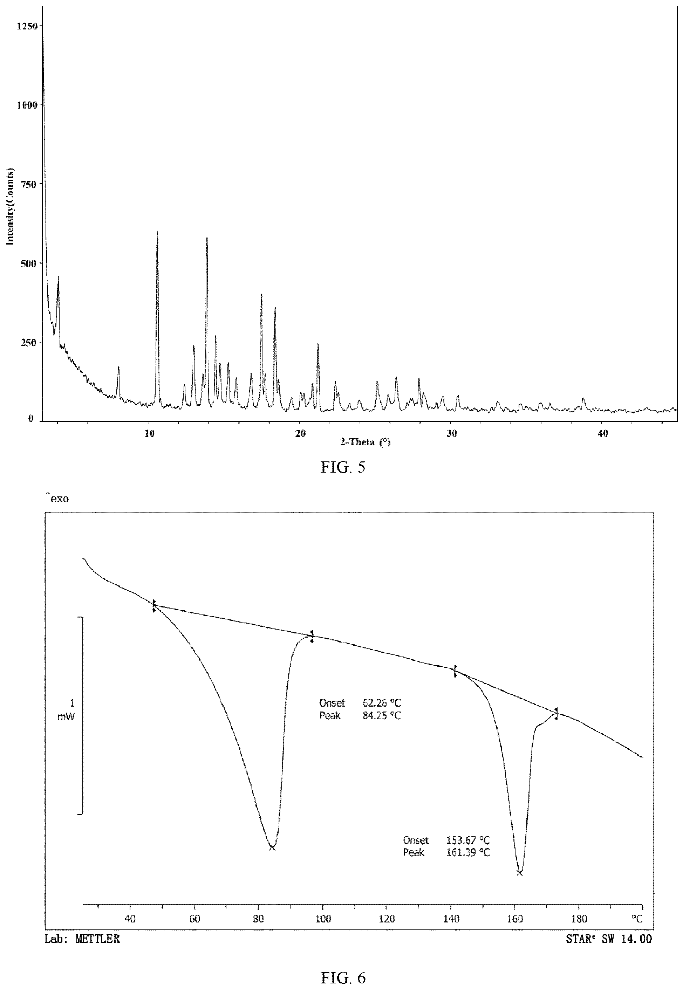

In one embodiment, the crystalline form II has an XRPD pattern substantially as shown in FIG. 5.

In one embodiment, the crystalline form II has a DSC profile comprising endothermic peaks at about 78-90 °C and about 160-162 °C, for example, endothermic peaks at about 82-86 °C and about 160-162 °C, for example, endothermic peaks at about 84 °C and about 161 °C.

In one embodiment, the crystalline form II has a DSC profile substantially as shown in FIG. 6.

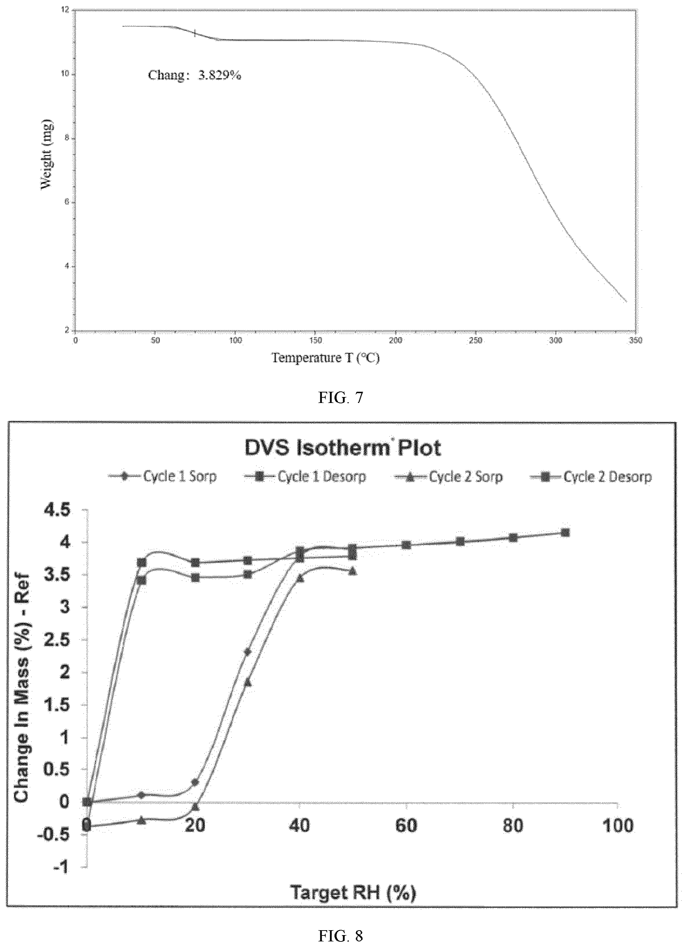

In one embodiment, the crystalline form II has a TGA profile substantially as shown in FIG. 7.

In one embodiment, the crystalline form II is a hydrate; preferably, the crystalline form II is a monohydrate.

In one embodiment, the crystalline form II has a DVS profile substantially as shown in FIG. 8.

In one embodiment, the crystalline form III of compound 2 has an X-ray powder diffraction pattern comprising characteristic peaks at one or more of 2θ angles of 3.4±0.2°, 9.8±0.2°, 12.1±0.2°, 13.1±0.2°, 16.4+0.2°, and 21.9+0.2°.

In one embodiment, the crystalline form III of compound 2 has an X-ray powder diffraction pattern comprising characteristic peaks at one or more of 2θ angles of 3.4+0.2°, 9.8+0.2°, 11.4+0.2°, 12.1±0.2°, 13.1+0.2°, 14.7+0.2°, 16.4+0.2°, 19.9+0.1°, and 21.9+0.2°.

In one embodiment, the crystalline form III has an X-ray powder diffraction pattern comprising diffraction peaks at the following 2θ angles:

| No. | 2θ±0.2° | d value | Relative intensity | No. | 2θ±0.2° | d value | Relative intensity |
|---|---|---|---|---|---|---|---|
| 1 | 3.39 | 26.01 | 100% | 7 | 13.33 | 6.64 | 20.5% |
| 2 | 6.61 | 13.37 | 6.3% | 8 | 14.71 | 6.02 | 28.4% |
| 3 | 9.84 | 8.98 | 30.8% | 9 | 16.36 | 5.41 | 30.6% |
| 4 | 11.36 | 7.78 | 24.2% | 10 | 16.88 | 5.25 | 22.3% |
| 5 | 12.12 | 7.29 | 45.4% | 11 | 19.93 | 4.45 | 28.3% |
| 6 | 13.05 | 6.78 | 44.7% | 12 | 21.85 | 4.07 | 37.5% |

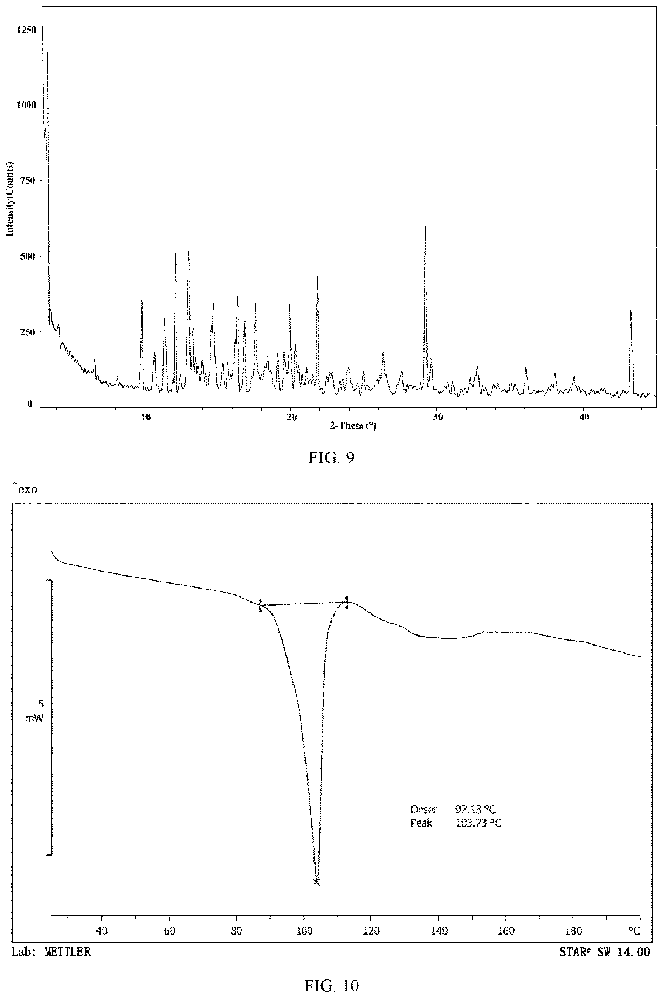

In one embodiment, the crystalline form III has an XRPD pattern substantially as shown in FIG. 9.

In one embodiment, the crystalline form III has a DSC profile comprising an endothermic peak at about 100-109 °C, for example, an endothermic peak at about 102-106 °C, for example, an endothermic peak at about 104 °C.

In one embodiment, the crystalline form III has a DSC profile substantially as shown in FIG. 10.

In one embodiment, the crystalline form III is a toluene solvate.

In one embodiment, the crystalline form IV of compound 2 has an X-ray powder diffraction pattern comprising characteristic peaks at one or more of 2θ angles of 10.7±0.2°, 11.4±0.2°, 12.9±0.2°, 13.3±0.2°, 16.1±0.2°, 17.6±0.2°, and 19.9±0.2°.

In one embodiment, the crystalline form IV of compound 2 has an X-ray powder diffraction pattern comprising characteristic peaks at one or more of 2θ angles of 10.7±0.2°, 11.4±0.2°, 12.1±0.2°, 12.9±0.2°, 13.3±0.2°, 16.1±0.2°, 17.6±0.2°, 19.1±0.2°, and 19.9±0.2°.

In one embodiment, the crystalline form IV has an X-ray powder diffraction pattern comprising diffraction peaks at the following 2θ angles:

| No. | 2θ±0.2° | d value | Relative intensity | No. | 2θ±0.2° | d value | Relative intensity |
|---|---|---|---|---|---|---|---|
| 1 | 4.11 | 21.50 | 27.2% | 7 | 12.94 | 6.84 | 93.7% |
| 2 | 8.36 | 10.56 | 8.0% | 8 | 13.27 | 6.67 | 88.0% |
| 3 | 9.70 | 9.11 | 21.5% | 9 | 16.11 | 5.50 | 53.0% |
| 4 | 10.69 | 8.27 | 65.9% | 10 | 17.60 | 5.03 | 100% |
| 5 | 11.44 | 7.73 | 63.3% | 11 | 19.06 | 4.65 | 47.9% |
| 6 | 12.05 | 7.34 | 46.4% | 12 | 19.85 | 4.47 | 55.6% |

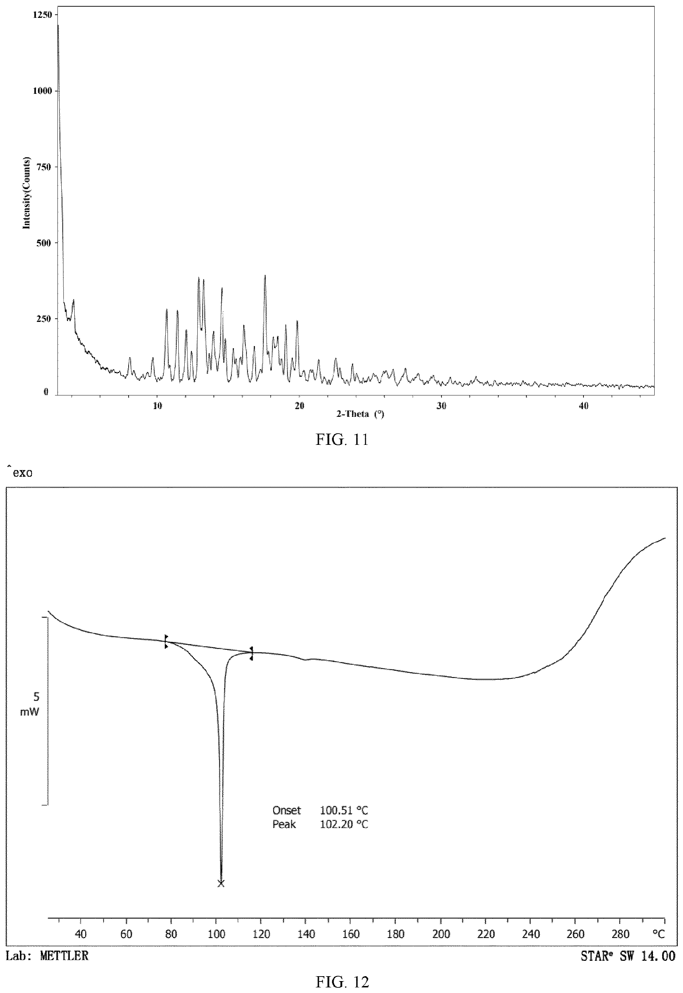

In one embodiment, the crystalline form IV has an XRPD pattern substantially as shown in FIG. 11.

In one embodiment, the crystalline form IV has a DSC profile comprising an endothermic peak at about 101-106 °C, for example, an endothermic peak at about 102 °C.

In one embodiment, the crystalline form IV has a DSC profile substantially as shown in FIG. 12.

In one embodiment, the crystalline form IV is a xylene solvate.

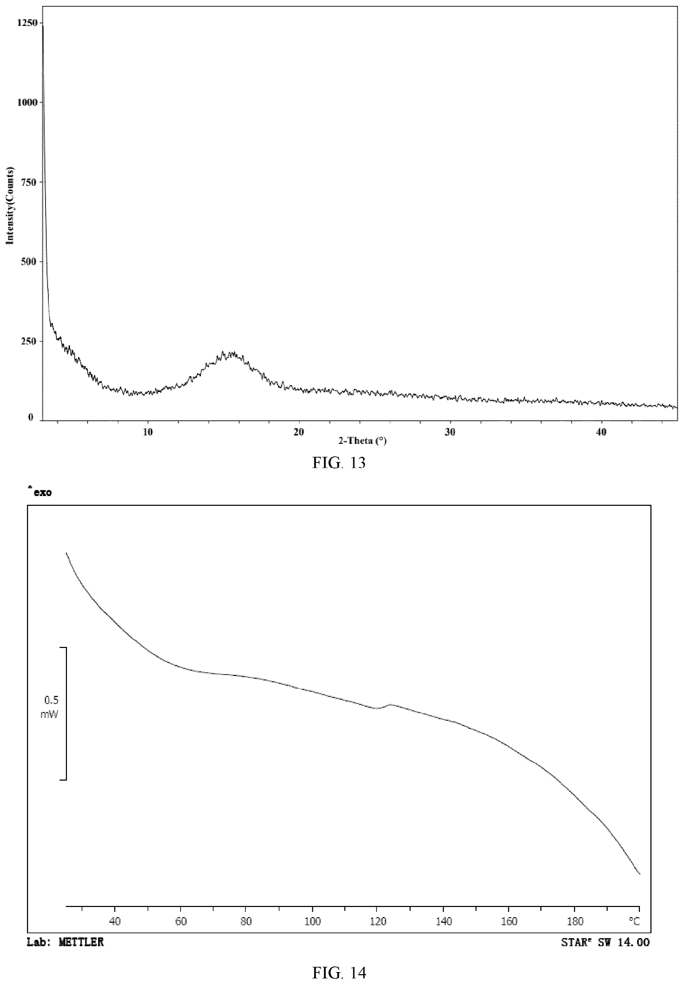

In one embodiment, the amorphous form of compound 2 has an XRPD pattern substantially as shown in FIG. 13.

In one embodiment, the amorphous form has a DSC profile substantially as shown in FIG. 14.

The present disclosure further provides a method for preparing a polymorph of compound 2.

In one embodiment, the crystalline form I of compound 2 is prepared by the following method: adding compound 2 to isopropyl acetate, heating and stirring, filtering, and drying to give the crystalline form I. Preferably, the weight-volume ratio of compound 2 to isopropyl acetate is 1 g:(5-30) mL, for example, 1 g:(5-20) mL, for example, 1 g:10 mL. The heating is performed at a temperature of 40-70 °C, for example, 45-55 °C, for example, 50 °C.

In one embodiment, the crystalline form II of compound **2** is prepared by the following method: adding compound **2** to water, heating for dissolution, and cooling for crystallization to give the crystalline form II. Preferably, the weight-volume ratio of compound **2** to water is 1 g:(40-100) mL, for example, 1 g:(40-60) mL, for example, 1 g:50 mL.

In one embodiment, the crystalline form III of compound **2** is prepared by the following method: adding compound **2** to toluene, heating for dissolution, and cooling for crystallization to give the crystalline form III. Preferably, the weight-volume ratio of compound **2** to toluene is 1 g:(10-50) mL, for example, 1 g:(20-40) mL, for example, 1 g:25 mL.

In one embodiment, the crystalline form IV of compound **2** is prepared by the following method: adding compound **2** to xylene, stirring at room temperature, filtering, and drying to give the crystalline form IV. Preferably, the weight-volume ratio of compound **2** to xylene is 1g:(10-50) mL, for example, 1 g:(20-40) mL, for example, 1 g:25 mL. Preferably, the drying is performed at a temperature of 40-60 °C, for example, 50 °C.

In one embodiment, the amorphous form of compound **2** is prepared by the following method: adding compound **2** to dichloromethane, stirring for dissolution, filtering, and concentrating the filtrate to dryness under reduced pressure to give the amorphous form. Preferably, the weight-volume ratio of compound **2** to dichloromethane is 1 g:(10-30) mL, for example, 1 g:(10-15) mL, for example, 1 g:20 mL. The concentration under reduced pressure is performed at a temperature of 30-45 °C, for example, 35-40 °C.

In the present disclosure, in the method for preparing the polymorph described above, the cooling for crystallization may be performed at room temperature or below, for example, at 0-25 °C, for example, at 0-10 °C. The method may further comprise steps of filtration and drying, which may be performed according to conventional procedures. For example, drying in vacuum is performed at 40-70 °C, for example, at 50-60 °C.

The crystal prepared in the present disclosure has a purity of greater than 95%, preferably a purity of greater than 97%, more preferably a purity of greater than 99%, and most preferably a purity of greater than 99.5%.

The present disclosure further provides a pharmaceutical composition, comprising at least one of the pharmaceutically acceptable salt of compound **1,** and the crystalline form or the amorphous form thereof (e.g., the crystalline form of compound **1** hydrochloride) described herein, and optionally a pharmaceutically acceptable carrier.

According to an embodiment of the present disclosure, the crystalline form is selected from at least one of the crystalline forms I, II, III, and IV of compound **2** described above.

According to an embodiment of the present disclosure, the amorphous form is the amorphous form of compound **2.**

The present disclosure further provides use of at least one of the pharmaceutically acceptable salt of compound **1,** and the crystalline form or the amorphous form thereof (for example, the crystalline form of compound **1** hydrochloride), or the pharmaceutical composition described above for manufacturing a medicament for the prevention and/or treatment of central nervous system diseases, Alzheimer's disease, epilepsy, or depression, particularly postpartum depression, or for sedation and hypnosis.

According to an embodiment of the present disclosure, the central nervous system diseases are, for example, traumatic brain injury, essential tremor, epilepsy (including refractory persistent epilepsy), rare genetic epilepsy (e.g., Dravet syndrome and Rett syndrome), depression (including postpartum depression), and Alzheimer's disease. The central nervous system diseases are selected from, for example, essential tremor, epilepsy, clinical depression, postnatal or postpartum depression, atypical depression, psychotic major depression, catatonic depression, seasonal affective disorder, dysthymia, double depression, depressive personality disorder, recurrent transient depression, minor depressive disorder, bipolar disorder or manic depressive disorder, post-traumatic stress disorder, depression caused by chronic medical conditions, treatment-resistant depression, refractory depression, suicidal tendency, suicidal ideations, and suicidal behaviors.

The present disclosure provides various salt forms of an allopregnanolone derivative, including hydrochloride, phosphate, *L*-malate, maleate, *p*-toluenesulfonate, benzoate, and the like. The present disclosure further provides a plurality of crystalline forms and amorphous form of the allopregnanolone derivative hydrochloride, including crystalline form I, crystalline form II, crystalline form III, and crystalline form IV. The allopregnanolone derivative hydrochloride, and the crystalline form I thereof and phosphate have good stability, and the preparation method possesses ease to operate, suitability for scale-up, and good industrial application prospects.

### BRIEF DESCRIPTION OF THE DRAWINGS

FIG. 1 is an XRPD pattern of crystalline form I of compound **2** of the present disclosure;
FIG. 2 is a DSC profile of crystalline form I of compound **2** of the present disclosure;
FIG. 3 is a TGA profile of crystalline form I of compound **2** of the present disclosure;
FIG. 4 is a DVS profile of crystalline form I of compound **2** of the present disclosure;
FIG. 5 is an XRPD pattern of crystalline form II of compound **2** of the present disclosure;
FIG. 6 is a DSC profile of crystalline form II of compound **2** of the present disclosure;
FIG. 7 is a TGA profile of crystalline form II of compound **2** of the present disclosure;
FIG. 8 is a DVS profile of crystalline form II of compound **2** of the present disclosure;
FIG. 9 is an XRPD pattern of crystalline form III of compound **2** of the present disclosure;
FIG. 10 is a DSC profile of crystalline form III of compound **2** of the present disclosure;
FIG. 11 is an XRPD pattern of crystalline form IV of compound **2** of the present disclosure;
FIG. 12 is a DSC profile of crystalline form IV of compound **2** of the present disclosure;
FIG. 13 is an XRPD pattern of an amorphous form of compound **2** of the present disclosure; and
FIG. 14 is a DSC profile of an amorphous form of compound **2** of the present disclosure.

### DETAILED DESCRIPTION

The present disclosure will be described in detail below by the following examples. It will be appreciated that the methods in the examples are for illustrative purposes only and do not constitute any limitation to the present disclosure. Unless otherwise specified, the materials used in the examples are all commercially available or prepared according to known or conventional methods. Unless otherwise specified, the experimental methods used are all conventional methods.

### Instruments and Methods:

XRPD (X-ray powder diffraction): instrument: Bruker D8 ADVANCE X-ray diffractometer; XRPD parameters: light tube: Cu, kα, (λ = 1.54056 Å); light tube voltage: 40 kV, light tube current: 40 mA; scanning range: 3-45 deg; step size: 0.02 deg; scanning time: 0.12 s. Procedure: A sample of about 10-20 mg is subjected to XRPD detection.

DSC (differential scanning calorimetry): instrument: METTLER TOLEDO DSC3+ differential scanning calorimeter; DSC parameters: temperature range: 25-300 °C; heating rate: 10 °C/min; nitrogen purge gas: 50 mL/min. Procedure: A sample (3-5 mg) is placed in a DSC aluminum pan and tested.

TGA (thermogravimetric analysis): instrument: TA TGA550, USA; TGA parameters: temperature range: 30-300 °C; heating rate: 10 °C/min; nitrogen purge gas: 25 mL/min. Procedure: A sample (5-10 mg) is placed in a TGA platinum pan and tested.

Table 1 below is the definition and range of hygroscopicity for a drug after equilibration at 25 °C ± 1 °C and 80% ± 2% RH in Guidelines for Hygroscopicity Trials for Pharmaceuticals (Chinese Pharmacopoeia, 2020 Edition, Volume IV, page 485).

**Table 1**

| Deliquescence | Absorbing sufficient water to form a solution |
|---|---|
| Very hygroscopic | Hygroscopic weight gain being not less than 15% |
| Hygroscopic | Hygroscopic weight gain being less than 15% but not less than 2% |
| Slightly hygroscopic | Hygroscopic weight gain being less than 2% but not less than 0.2% |
| Non-hygroscopic or hardly hygroscopic | Hygroscopic weight gain being less than 0.2% |

### Materials:

As described below, compound **1** or the hydrochloride thereof (compound **2**) used in the examples can be manufactured according to the method of Example 1 in Chinese Patent Application No. CN202211112060.3, the content of which is incorporated herein by reference in its entirety. Compound **2** can also be prepared using the salification method of the present disclosure.

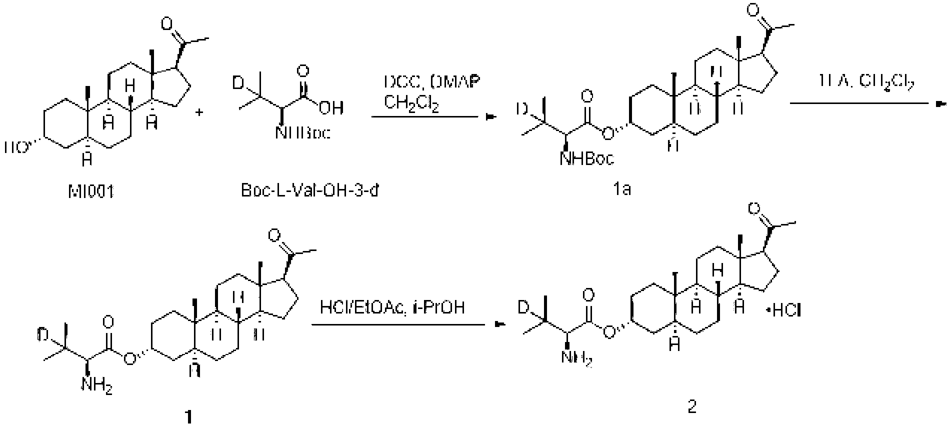

### Synthesis of intermediate 1a:

Boc-L-Val-OH-3-d (1.20 g, 5.5 mmol; see CN113461573A), MI001 (1.91 g, 6.0 mmol), and 4-dimethylaminopyridine (0.07 g, 0.6 mmol) were dissolved in dichloromethane (15 mL), and a solution of dicyclohexylcarbodiimide (1.24 g, 6.0 mmol) in dichloromethane (5 mL) was added dropwise at 20 °C. The mixture was stirred overnight and filtered to remove dicyclohexylurea. The filtrate was concentrated and separated by column chromatography (V_{petroleum ether/ethyl acetate} was 20:1-7:1) to give intermediate **1a** as a colorless oil (80% yield). ¹H NMR (400 MHz, CDCl₃) δ 5.10 (m, 1H), 5.07 (d, J = 9.5 Hz, 1H), 4.23 (d, J = 9.1 Hz, 1H), 2.53 (t, J = 8.8 Hz, 1H), 2.26-2.08 (m, 1H), 2.11 (s, 3H), 2.06-1.95 (m, 1H), 1.85-0.71 (m, 20H), 1.46 (s, 9H), 0.98 (s, 3H), 0.90 (s, 3H), 0.80 (s, 3H), 0.61 (s, 3H).

### Synthesis of compound 1:

Intermediate **1a** (2.3 g, 4.4 mmol) was dissolved in dichloromethane (15 mL) and stirred at 20 °C for dissolution, and trifluoroacetic acid (5.02 g, 44.0 mmol) was added dropwise. The mixture was stirred for 3-4 h while the temperature was controlled at 15-25 °C, and dichloromethane (20 mL) was added. The reaction solution was slowly decanted into an aqueous sodium bicarbonate solution (15 g/50 mL) under stirring. After stirring for 5-15 min, the mixture was left to stand for phase separation. The organic phase was washed with purified water (50 mL), dried over anhydrous sodium sulfate, filtered, and concentrated to give compound **1** (1.73 g).

### Synthesis of compound 2:

Compound **1** (1.59 g, 3.8 mmol) was dissolved in ethyl acetate (20 mL) and isopropanol (1.3 mL), and a solution of hydrogen chloride in ethyl acetate (2.4 M, 1.6 mL, 3.8 mmol) was added dropwise. The mixture was stirred at 20 °C for 1 h and filtered. The filter cake was washed with ethyl acetate (20 mL) and dried at 40 °C in vacuum by an oil pump (P ≤ -0.09 MPa) for 4 h to give compound **2** as a white solid (68% yield).

### Example 1. Preparation of different salt forms of compound 1

About 750 mg of compound 1 was dissolved in a solvent of 15 mL, stirred for dissolution, and filtered through a 0.22 µm PTFE filter membrane to give a clear solution of compound **1.** 1 mL of the above solution (containing about 50 mg of compound **1**) was taken, and different acids were separately added for salification reactions. The precipitated solids were collected and dried in vacuum at 40 °C to give different salt forms of compound **1.** The conditions of the salification reaction are shown in Table 2.

**Table 2. Preparation of different salt forms of compound 1**

| Experiment No. | Solvent | Acid | Compound **1**:acid molar ratio | System volume (mL) | Product |
|---|---|---|---|---|---|
| 1 | Acetonitrile | Hydrochloric acid | 1:1 | 2.5 | White powder |
| 2 | Ethyl acetate | Phosphoric acid | 1:0.5 | 1 | White powder |
| 3 | Ethyl acetate | Acetic acid | 1:1 | 1 | White powder |
| 4 | Acetonitrile | *L*-Malic acid | 1:0.5 | 2.5 | White powder |
| 5 | Ethyl acetate | Maleic acid | 1:0.5 | 2 | White powder |
| 6 | Acetonitrile | *p*-Toluenesulfonic acid | 1:1 | 2.5 | White powder |
| 7 | Ethyl acetate | Benzoic acid | 1:1 | 2 | White powder |
| 8 | Acetonitrile | Benzenesulfonic acid | 1:1 | 2.5 | Gum |

The ¹H-NMR data of different salt forms are as follows:
Compound **1** hydrochloride: ¹H NMR (400 MHz, CDCl₃) δ 8.83 (brs, 3H), 5.23-5.14 (m, 1H), 4.00-3.88 (m, 1H), 2.52 (t, J = 8.7 Hz, 1H), 2.22-2.08 (m, 1H), 2.11 (s, 3H), 2.06-1.96 (m, 1H), 1.86-1.08 (m, 18H), 1.18 (s, 3H), 1.17 (s, 3H), 1.04-0.88 (m, 1H), 0.86-0.71 (m, 1H), 0.80 (s, 3H), 0.61 (s, 3H).
Compound **1** phosphate: ¹H NMR (400 MHz, DMSO-d₆) δ 6.98 (brs, 5H), 5.13 (s, 1H), 3.97 (s, 1H), 2.52 (t, , 1H), 2.15 (d, 3H), 2.11 (s, 2H), 2.01 (d, 1H), 1.76 (d, 1H), 1.66 (s, 3H), 1.54 (m, 7H), 1.45 (m, 2H), 1.28 (d, 1H), 1.12 (m, 8H), 0.98 (s, 3H), 0.79 (s, 3H), 0.61 (s, 3H).
Compound **1** acetate: ¹H NMR (400 MHz, CDCl₃) δ 5.13-5.05 (m, 1H), 3.65 (brs, 3H), 3.35 (s, 1H), 2.52 (t, J = 8.9 Hz, 1H), 2.22-2.09 (m, 1H), 2.11 (s, 3H), 2.05-1.96 (m, 1H), 2.07 (s, 3H), 1.79-0.87 (m, 19H), 1.00 (s, 3H), 0.92 (s, 3H), 0.86-0.72 (m, 1H), 0.80 (s, 3H), 0.61 (s, 3H).
Compound **1** *L*-malate: ¹H NMR (400 MHz, DMSO-d₆) δ 6.24 (brs, 4H), 5.14 (s, 1H),4.31 (t, 1H), 3.90 (s, , 1H), 2.77 (s, 2H), 2.52(d,1H)2.15(s, 1H), 2.11 (s, 3H), 2.04(s,2H),1.50 (d, 1H), 1.42 (m,1H), 1.24 (s, 3H), 1.19 (m, 6H), 1.07 (d, 6H), 0.96 (s, 3H), 0.80 (s, 3H).0.61(s,3H).
Compound **1** maleate: ¹H NMR (400 MHz, CDCl₃) δ 6.23 (s, 1H), 5.25-5.03 (m, 1H), 3.69 (s, 1H), 2.52 (t, J = 8.9 Hz, 1H), 2.23-2.09 (m, 1H), 2.11 (s, 3H), 2.04-1.97 (m, 1H), 1.82-0.90 (m, 19H), 1.06 (s, 3H), 1.02 (s, 3H), 0.86-0.72 (m, 1H), 0.80 (s, 3H), 0.61 (s, 3H).
Compound **1** *p*-toluenesulfonate: ¹H NMR (400 MHz, CDCl₃) δ 8.30-8.05 (m, 3H), 7.77 (d, J = 8.0 Hz, 2H), 7.14 (d, J = 7.9 Hz, 2H), 5.12-5.05 (m, 1H), 3.92-3.83 (m, 1H), 2.48 (t, J = 8.7 Hz, 1H), 2.35 (s, 3H), 2.22-2.06 (m, 1H), 2.10 (s, 3H), 2.01-1.91 (m, 1H), 1.77-0.82 (m, 19H), 1.01 (s, 3H), 0.98 (s, 3H), 0.81-0.69 (m, 1H), 0.76 (s, 3H), 0.59 (s, 3H).
Compound **1** benzoate: ¹H NMR (400 MHz, CDCl₃) δ 8.13-8.06 (m, 2H), 7.62-7.53 (m, 1H), 7.50-7.40 (m, 1H), 5.16-5.05 (m, 1H), 4.09 (brs, 3H), 3.41 (s, 1H), 2.51 (t, J = 8.9 Hz, 1H), 2.22-2.10 (m, 1H), 2.11 (s, 3H), 2.03-1.96 (m, 1H), 1.77-0.86 (m, 19H), 1.02 (s, 3H), 0.94 (s, 3H), 0.83-0.71 (m, 1H), 0.80 (s, 3H), 0.61 (s, 3H).
Compound **1** benzenesulfonate: ¹H NMR (400 MHz, CDCl₃) δ 8.22-8.01 (m, 3H), 7.93-7.82 (m, 2H), 7.42-7.29 (m, 3H), 5.12-5.03 (m, 1H), 3.99-3.87 (m, 1H), 2.49 (t, J = 8.7 Hz, 1H), 2.22-2.06 (m, 1H), 2.10 (s, 3H), 2.02-1.96 (m, 1H), 1.76-0.82 (m, 19H), 1.02 (s, 3H), 1.00 (s, 3H), 0.81-0.68 (m, 1H), 0.76 (s, 3H), 0.59 (s, 3H).

### Example 2. Study on properties of different salt forms

In this example, the hygroscopicity, solubility, and chemical stability of different salt forms of compound **1** obtained in Example 1 were investigated.

### 2.1 Hygroscopicity

The strong hygroscopicity of a drug will seriously affect the flowability of the drug and even affect the stability of the drug. Therefore, according to the present application, a hygroscopic weight gain experiment was conducted on different salt forms of compound **1** as per the method described in Guidelines for Hygroscopicity Trials for Pharmaceuticals (Chinese Pharmacopoeia, 2020 Edition, Volume IV). The results of the hygroscopicity test are shown in Table 3.

**Table 3. Hygroscopicity test results of different salt forms**

| Sample No. | Salt form | Hygroscopic weight gain | Description of hygroscopicity |
|---|---|---|---|
| 1 | Hydrochloride | 0.2% | Slightly hygroscopic |
| 2 | Phosphate | 4.9% | Hygroscopic |
| 3 | Acetate | 0.4% | Slightly hygroscopic |
| 4 | *L*-Malate | 4.0% | Hygroscopic |
| 5 | Maleate | 0% | Non-hygroscopic |
| 6 | *p*-Toluenesulfonate | 0% | Non-hygroscopic |
| 7 | Benzoate | 0.1% | Non-hygroscopic |
| 8 | Benzenesulfonate | 0.2% | Slightly hygroscopic |

As can be seen from the hygroscopicity results, except that the phosphate and the L-malate were hygroscopic, other salt forms were slightly hygroscopic or non-hygroscopic. Therefore, the salt forms described above are all suitable for drug research and development.

### 2.2 Solubility

The free base of compound **1** and different salts thereof were separately added to 8 parts of purified water of 4 mL until the solution was saturated, and the corresponding amount was recorded. The system was stirred in a water bath at 25 °C. In addition, the same system was prepared by the same method and stirred at 37 °C. After 24 h, the supernatant was taken for HPLC analysis. The solubility results of different salt forms in water are shown in Table 4.

**Table 4. Solubility of different salt forms in water**

| Compound | Solubility at 25 °C (mg/mL) | Solubility at 37 °C (mg/mL) | Compound | Solubility at 25 °C (mg/mL) | Solubility at 37 °C (mg/mL) |
|---|---|---|---|---|---|
| Free base | 0.01 | Not detected | Maleate | 0.73 | 1.12 |
| Hydrochloride | 22.74 | 46.39 | *p*-Toluenesulfonate | 0.81 | 0.89 |
| Phosphate | 6.93 | 14.29 | Benzoate | 0.15 | 0.16 |
| Acetate | 1.22 | 1.60 | Benzenesulfonate | 1.51 | 1.57 |
| *L*-Malate | 1.40 | 2.11 | - | - | - |

From the solubility investigation results, it can be seen that the salt forms of compound **1** exhibited improved solubility as compared to the free base; among them, the hydrochloride and the phosphate salt had higher solubility, and the hydrochloride had the highest solubility in water.

### 2.3 Chemical stability

Appropriate amounts of different salts of compound **1** were left to stand open to investigate the stability of samples under the conditions of high temperature (60 °C), high humidity (RH 92.5%), and illumination (4500±500 Lux). Samples were taken separately at three time points, day 0, day 7, and day 14, for HPLC purity analysis. The results are shown in Table 5.

**Table 5. Chemical stability of different salt forms**

| Salt form | Time (day) | High temperature/60 °C | High humidity/RH 92.5% | Illumination/4500±500 Lux |
|---|---|---|---|---|
| Hydrochloride | 0 | 100% | 100% | 100% |
| | 7 | 99.89% | 99.89% | 99.89% |
| | 14 | 99.88% | 99.89% | 99.89% |
| Phosphate | 0 | 99.89% | 99.89% | 99.89% |
| | 7 | 98.49% | 99.86% | 99.74% |
| | 14 | 98.19% | 99.76% | 99.75% |
| Acetate | 0 | 88.52% | 88.52% | 88.52% |
| | 7 | 82.15% | 85.66% | 84.61% |
| | 14 | 84.95% | 84.94% | 84.94% |
| *L*-Malate | 0 | 99.34% | 99.34% | 99.34% |
| | 7 | 97.91% | 99.48% | 99.27% |
| | 14 | 96.93% | 99.33% | 98.91% |
| Maleate | 0 | 99.26% | 99.26% | 99.26% |
| | 7 | 98.54% | 99.72% | 99.37% |
| | 14 | 97.25% | 99.66% | 98.79% |
| *p*-Toluenesulfonate | 0 | 98.81% | 98.81% | 98.81% |
| | 7 | 98.35% | 98.96% | 98.59% |
| | 14 | 97.87% | 99.00% | 98.33% |
| Benzoate | 0 | 99.55% | 99.55% | 99.55% |
| | 7 | 98.91% | 99.44% | 99.40% |
| | 14 | 97.64% | 99.34% | 99.38% |
| Benzenesulfonate | 0 | 93.97% | 93.97% | 93.97% |
| | 7 | 93.91% | 95.04% | 93.21% |
| | 14 | 93.13% | 93.71% | 92.63% |

As can be seen from the experimental results, compound **1** hydrochloride exhibited good stability; the phosphate, the benzoate, and the *p*-toluenesulfonate exhibited relatively good chemical stability, but the high temperature exhibited a certain influence on the stability of the three salt forms.

In summary, preferred pharmaceutically acceptable salts of compound **1** include hydrochloride, phosphate, benzoate, and *p*-toluenesulfonate, more preferably hydrochloride or phosphate, and most preferably hydrochloride.

### Example 3. Scale-up preparation of hydrochloride and phosphate of compound 1

### 3.1 Preparation of compound 1 hydrochloride

In a nitrogen atmosphere, 14 g of compound **1,** 210 mL of ethyl acetate (15 V), and 14 mL of isopropanol (1 V) were added into a three-necked flask, and stirred for dissolving. 2 mol/L solution of hydrogen chloride in ethyl acetate was added dropwise to adjust the pH to 3-4, and crystallization was performed for 1 h. Then the mixture was cooled to 0-5 °C for crystallization for 2-3 h and filtered. The filter cake was washed with ethyl acetate and dried under reduced pressure at 50 °C to give compound **1** hydrochloride as a white solid (9.5 g).

### 3.2 Preparation of phosphate of compound 1

10 g of compound **1** and 150 mL of ethyl acetate were added into a three-necked flask, and stirred for dissolving at 40 °C. A solution of phosphoric acid in ethyl acetate was added dropwise to adjust the pH to 3-4, and crystallization was performed for 1 h. Then the mixture was cooled to 0-5 °C for crystallization for 2-3 h and filtered. The filter cake was washed with ethyl acetate and dried under reduced pressure at 50 °C to give the phosphate of compound **1** as a white solid.

### Example 4. Preparation of polymorphs of compound 1 hydrochloride (i.e., compound 2)

In order to develop solid formulations of the compound, in the present disclosure, compound **2** was further subjected to polymorph screening tests to give four crystalline forms I, II, III, and IV of compound **2** and one amorphous form.

Exemplary preparation examples of the polymorphs of the compound are provided below, where the stability of the polymorphs was tested.

### 4.1 Preparation of crystalline form I of compound 2

About 5.0 g of compound **2** was added to 50 mL of isopropyl acetate, stirred at 50 °C for 12 h and filtered. The filter cake was washed with 10 mL of isopropyl acetate and dried in vacuum at 50 °C to give the crystalline form I of compound **2.**

The XRPD pattern of the resulting crystalline form I is substantially as shown in FIG. 1. The DSC profile is substantially as shown in FIG. 2. The TGA profile is substantially as shown in FIG. 3. The DVS profile is shown in FIG. 4. The DSC profile of the crystalline form I shows a single strong endothermic peak at about 156 °C, which is a melting peak, while no other thermal phenomena are found in the profile. The TGA profile shows no significant weight loss plateau and a weight loss of 1.40% (adsorbed water), indicating that the crystalline form I is an anhydrous crystalline form. The DVS profile shows that the sample began to absorb moisture and gain weight when the humidity increased from 0-20%, and the weight gain was 1.2% when the relative humidity was 90%.

### 4.2 Preparation of crystalline form II of compound 2

About 2.0 g of compound **2** was added to 100 mL of purified water, and stirred at 60 °C for dissolving. The solution was filtered through a 0.22 µm PTFE filter membrane. The filtrate was heated to 60 °C, stirred, cooled to room temperature for crystallization, and filtered. The filter cake was dried in vacuum at 50 °C to give the crystalline form II of compound **2.**

The XRPD pattern of the resulting crystalline form II is substantially as shown in FIG. 5. The DSC profile is substantially as shown in FIG. 6. The TGA profile is substantially as shown in FIG. 7. The DVS profile is shown in FIG. 8. The DSC profile of the crystalline form II shows endothermic peaks at about 84.25 °C and 161.39 °C. The TGA profile shows a significant thermal weight loss plateau and a weight loss of 3.829%, indicating that the crystalline form II is a monohydrate. The DVS profile shows that the sample began to absorb moisture and gain weight when the humidity increased from 20%, and the weight gain was about 4% when the relative humidity was 50%.

### 4.3 Preparation of crystalline form III of compound 2

About 2.0 g of compound **2** was added to 50 mL of toluene, and stirred for dissolving. The solution was filtered through a 0.22 µm PTFE filter membrane. The filtrate was slowly evaporated in an open flask at room temperature. After a solid was precipitated, the solid was collected. The solid was dried in vacuum at 50 °C to give the crystalline form III of compound **2.**

The XRPD pattern of the resulting crystalline form III is substantially as shown in FIG. 9. The DSC profile is substantially as shown in FIG. 10. The DSC profile of the crystalline form III shows a single strong endothermic peak at 103.73 °C, which is a desolvation peak, demonstrating that the crystalline form is a toluene solvate.

### 4.4 Preparation of crystalline form IV of compound 2

2.0 g of compound **2** was added to 50 mL of xylene, stirred at room temperature for 24 h and filtered. The filter cake was rinsed with a small amount of xylene and dried in vacuum at 50 °C to give the crystalline form IV of compound **2.**

The XRPD pattern of the resulting crystalline form IV is substantially as shown in FIG. 11. The DSC profile is substantially as shown in FIG. 12. The DSC profile of the crystalline form IV shows a single strong endothermic peak at 102.20 °C, which is a desolvation peak, demonstrating that the crystalline form is a xylene solvate.

### 4.5 Preparation of amorphous form of compound 2

About 2.0 g of compound **2** was added to 40 mL of dichloromethane solvent, and stirred for dissolving. The solution was filtered through a 0.22 µm PTFE filter membrane. The filtrate was concentrated to dryness under reduced pressure at 35 °C to give a white solid. The resulting white solid was dried in vacuum to give the amorphous form of compound **2.**

The XRPD pattern of the resulting amorphous form is substantially as shown in FIG. 13, with diffusion peaks and no significant characteristic diffraction angles. The DSC profile is substantially as shown in FIG. 14, where no significant endothermic and exothermic phenomena are observed.

### Example 5. Polymorph conversion study of compound 2

Interconversion may occur between different crystalline forms of the same compound, and therefore, a drug prepared from a certain crystalline form may be converted into another crystalline form during storage, which may affect the efficacy of the drug and even cause adverse effects. Therefore, in this example, the interconversion relationship between the crystalline forms of compound **2** was investigated to find a stable crystalline form.

Samples of two different crystalline forms were mixed and dissolved in a corresponding solvent of 5 mL, and the mixture was stirred at room temperature for 3 days and separated by centrifugation. The resulting solid was dried in vacuum at 40 °C. The dried product was subjected to a DSC test to determine the composition of the crystalline form. The experimental scheme and results are shown in Table 6.

**Table 6. Study on interconversion between crystalline forms**

| Experiment No. | Sample | Solvent | Volume | Time | Final crystalline form |
|---|---|---|---|---|---|
| 1 | 100 mg of crystalline form I + 100 mg of crystalline form II | Isopropyl acetate | 5 ml | 3 days | I |
| 2 | 100 mg of crystalline form I + 100 mg of crystalline form II | n-Heptane | 5 ml | 3 days | I+II |
| 3 | 40 mg of crystalline form I + 40 mg of crystalline form III | Isopropyl acetate | 5 ml | 3 days | I |
| 4 | 40 mg of crystalline form I + 40 mg of crystalline form III | n-Heptane | 5 ml | 3 days | I+II |
| 5 | 40 mg of crystalline form I + 40 mg of crystalline form IV | Isopropyl acetate | 5 ml | 3 days | I |
| 6 | 40 mg of crystalline form I + 40 mg of crystalline form IV | n-Heptane | 5 ml | 3 days | I |
| 7 | 40 mg of crystalline form II + 40 mg of crystalline form III | Isopropyl acetate | 5 ml | 3 days | I |
| 8 | 40 mg of crystalline form II + 40 mg of crystalline form III | n-Heptane | 5 ml | 3 days | II |
| 9 | 40 mg of crystalline form II + 40 mg of crystalline form IV | Isopropyl acetate | 5 ml | 3 days | I |
| 10 | 40 mg of crystalline form II + 40 mg of crystalline form IV | n-Heptane | 5 ml | 3 days | II |
| 11 | 40 mg of crystalline form III + 40 mg of crystalline form IV | Isopropyl acetate | 5 ml | 3 days | I |
| 12 | 40 mg of crystalline form III + 40 mg of crystalline form IV | n-Heptane | 5 ml | 3 days | II |

The experimental results show that the crystalline form II, the crystalline form III, and the crystalline form IV were easily dehydrated and desolvated in the isopropyl acetate solvent system and were converted into the crystalline form I; the crystalline form III and the crystalline form IV were easily desolvated in the *n*-heptane system and were converted into the crystalline form II. In general, the crystalline form I and the crystalline form II have better stability than the crystalline form III and the crystalline form IV.

### Example 6. Stability investigation of crystalline form I and crystalline form II of compound 2

In this example, the crystalline form I and the crystalline form II of compound **2** were further subjected to physicochemical stability studies. Samples of the crystalline form I and the crystalline form II of compound **2** (about 50 mg) were separately taken and left to stand flat to investigate the stability under the conditions of high temperature (60 °C), high humidity (RH 92.5%), and illumination. Samples were taken on day 0, day 10, and day 30 for XRPD and DSC detections to evaluate the physical stability and for HPLC purity detection to evaluate the chemical stability. The results are shown in Table 7.

**Table 7. Stability results for crystalline form I and crystalline form II**

| Sample | Time (day) | High temperature (60 °C) | | High humidity (RH 92.5%) | | Illumination (4500±500 Lux) | |
|---|---|---|---|---|---|---|---|
| | | Purity | Crystalline form | Purity | Crystalline form | Purity | Crystalline form |
| Crystalline form I | 0 | 99.77% | I | 99.77% | Crystalline form I | 99.77% | I |
| | 5 | 99.78% | I | 99.72% | Crystalline form I | 99.78% | I |
| | 10 | 99.77% | I | 99.82% | Crystalline form I | 99.82% | I |
| | 30 | 99.64% | I | 99.78% | Crystalline form I | 99.65% | I |
| Crystalline form II | 0 | 99.59% | II | 99.59% | Crystalline form II | 99.59% | II |
| | 5 | 99.28% | Mixed crystal | 99.39% | Crystalline form II | 99.57% | II |
| | 10 | 98.93% | Mixed crystal | 99.65% | Crystalline form II | 99.68% | II |
| | 30 | 97.03% | Mixed crystal | 99.37% | Crystalline form II | 99.20% | II |

The experimental results show that after the crystalline form I was left to stand for 30 days under the conditions of high temperature (60 °C), high humidity (RH 92.5%), and illumination, the crystalline form patterns/profiles were substantially the same as those on day 0, the purity of the sample was substantially kept unchanged, and the related substance did not change significantly, indicating that the crystalline form I had good physical stability and chemical stability. After the crystalline form II was left to stand for 30 days under the conditions of high humidity (RH 92.5%) and illumination, the purity of the sample and the related substance did not change significantly, indicating that the crystalline form II had good chemical stability. However, after the crystalline form II was left to stand for 30 days under the condition of high temperature (60 °C), the purity slightly decreased, and the crystalline form II changed into mixed crystal, indicating that the stability of the crystalline form I was superior to that of the crystalline form II.

In summary, through the comparison of the polymorphs described above, the crystalline form I and the crystalline form II of compound 2 are relatively stable crystalline forms and can be used for the development of pharmaceutical formulations. Among them, the crystalline form I is an anhydrous form with a melting point of about 154-158 °C. It is non-hygroscopic and less prone to interconversion between crystalline forms and has good chemical stability and physical stability under different accelerated test conditions. Therefore, it features ease of scale-up preparation and suitability for industrial production. The crystalline form I is the most preferred crystalline form. Although the amorphous form is less stable than the crystalline form I, it may also be used to prepare solid dispersion formulations.

The above examples are only illustrative of the technical solutions of the present disclosure, and are not intended to limit same. Although the present disclosure has been illustrated in detail in the foregoing examples, those of ordinary skill in the art will appreciate that based on the content disclosed in the present disclosure, modifications can be made to the technical solutions described in the foregoing examples or equivalent replacements can be made to some of the technical features; such modifications and replacements do not make the essence of the corresponding technical solutions depart from the present disclosure and still fall within the claimed scope of the present disclosure.

## Claims

1. A pharmaceutically acceptable salt of compound **1** shown below, wherein the pharmaceutically acceptable salt is a salt formed by compound **1** and an organic acid or inorganic acid, 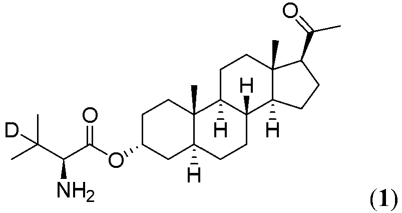 preferably, the pharmaceutically acceptable salt is selected from hydrochloride, phosphate, benzoate, *p*-toluenesulfonate, malate, acetate, benzenesulfonate, and maleate.

2. The salt according to claim 1, wherein in the hydrochloride, benzoate, and *p*-toluenesulfonate, the molar ratio of compound **1** to the acid is (1±0.1):1.

3. The salt according to claim 1, wherein in the phosphate, malate, and maleate, the molar ratio of compound **1** to the acid is (2±0.2):1.

4. A crystalline form I of compound **2,** wherein the crystalline form I has an X-ray powder diffraction pattern comprising characteristic peaks at one or more of 2θ angles of 6.3+0.2°, 10.9+0.2°, 13.3+0.2°, 13.5±0.2°, 15.1+0.2°, and 17.8+0.2°;
preferably, the crystalline form I has an X-ray powder diffraction pattern comprising characteristic peaks at one or more of 2θ angles of 6.3+0.2°, 10.9+0.2°, 13.3+0.2°, 13.5+0.2°, 14.2+0.2°, 15.1+0.2°, 15.8+0.2°, 17.4+0.2°, 17.8+0.2°, 20.9+0.2°, and 24.0+0.2°;
further preferably, the crystalline form I has an XRPD pattern substantially as shown in FIG. 1;

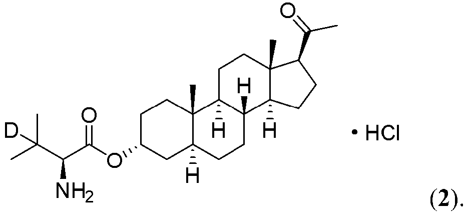

5. A crystalline form II of compound **2,** wherein the crystalline form II has an X-ray powder diffraction pattern comprising characteristic peaks at one or more of 2θ angles of 4.0±0.2°, 10.6±0.2°, 13.9±0.2°, 14.5±0.2°, 18.4±0.2°, and 21.3±0.2°;
preferably, the crystalline form II has an X-ray powder diffraction pattern comprising characteristic peaks at one or more of 2θ angles of 4.0±0.2°, 10.6±0.2°, 13.0±0.2°, 13.9±0.2°, 14.5±0.2°, 18.4±0.2°, and 21.3±0.2°;
further preferably, the crystalline form II has an XRPD pattern substantially as shown in FIG. 5;

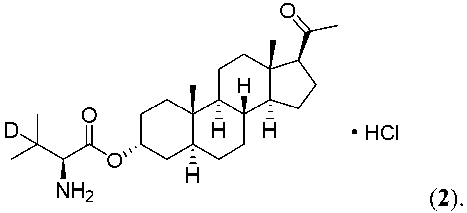

6. A crystalline form III of compound **2,** wherein the crystalline form III has an X-ray powder diffraction pattern comprising characteristic peaks at one or more of 20 angles of 3.4±0.2°, 9.8±0.2°, 12.1±0.2°, 13.1±0.2°, 16.4±0.2°, and 21.9±0.2°;
preferably, the crystalline form III has an X-ray powder diffraction pattern comprising characteristic peaks at one or more of 2θ angles of 3.4±0.2°, 9.8±0.2°, 11.4±0.2°, 12.1±0.2°, 13.1±0.2°, 14.7±0.2°, 16.4±0.2°, 19.9±0.2°, and 21.9±0.2°;
further preferably, the crystalline form III has an XRPD pattern substantially as shown in FIG. 9;

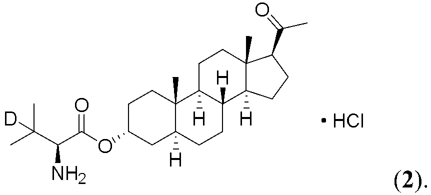

7. A crystalline form IV of compound **2,** wherein the crystalline form IV has an X-ray powder diffraction pattern comprising characteristic peaks at one or more of 2θ angles of 10.7±0.2°, 11.4±0.2°, 12.9±0.2°, 13.3±0.2°, 16.1±0.2°, 17.6±0.2°, and 19.9±0.2°;
preferably, the crystalline form IV has an X-ray powder diffraction pattern comprising characteristic peaks at one or more of 2θ angles of 10.7±0.2°, 11.4±0.2°, 12.0±0.2°, 12.9±0.2°, 13.3±0.2°, 16.1±0.2°, 17.6±0.2°, 19.1±0.2°, and 19.9±0.2°;
further preferably, the crystalline form IV has an XRPD pattern substantially as shown in FIG. 11;

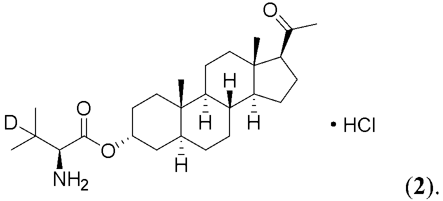

8. An amorphous form of compound **2,** wherein the amorphous form has an XRPD pattern substantially as shown in FIG. 13;

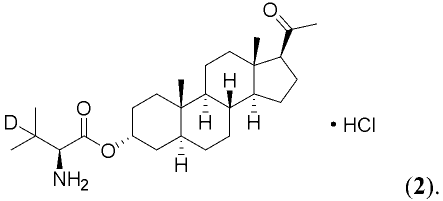

9. A pharmaceutical composition, comprising at least one of the pharmaceutically acceptable salt of compound **1** according to any one of claims 1 to 3, the crystalline form according to any one of claims 4 to 7, or the amorphous form according to claim 8, and optionally a pharmaceutically acceptable carrier.

10. Use of the pharmaceutically acceptable salt of compound **1** according to any one of claims 1 to 3, the crystalline form according to any one of claims 4 to 7, or the amorphous form according to claim 8 for manufacturing a medicament for the prevention and/or treatment of central nervous system diseases, Alzheimer's disease, epilepsy, or depression (e.g., postpartum depression), or for sedation and hypnosis.
